# EUROPEAN PATENT APPLICATION

(11) **EP 3 996 030 A1**
(43) Date of publication of application: **11.05.2022**
(21) Application number: 20206142.0
(22) Date of filing: 06.11.2020
(51) Int. Cl.: G06Q 50/20, G16H 80/00, G16H 20/00, G16H 10/00

(54) **USER INTERFACE SYSTEM FOR SELECTING LEARNING CONTENT**

(71) Applicant: Koninklijke Philips N.V., 5656 AG Eindhoven (NL)
(72) Inventor: DELLIMORE, Kiran Hamilton J., 5656 AE Eindhoven (NL); VAN GENUGTEN, Lenneke, 5656 AE Eindhoven (NL); SAINI, Privender Kaur, 5656 AE Eindhoven (NL); BOS, Colin, 5656 AE Eindhoven (NL)
(74) Representative: Philips Intellectual Property & Standards

(57) **Abstract**

A user interface system receives activity information relating at least to electronic device usage by a user. A mental state of the user is derived from this activity information, received during a preceding finite time period. Learning content is provided to a user based on learning goals and their associated difficulty levels, based on whether or not those goals have been met and also based on the mental state of the user. In this way, suitable learning content is selected based on the mental state of the user before the commencement of delivery of the learning content.

## Description

### FIELD OF THE INVENTION

This invention relates to user interaction with a system, such as an electronic health monitoring and training system. In particular, the invention relates to interaction with a system which delivers learning content for a user, with learning targets or goals.

### BACKGROUND OF THE INVENTION

There are many types of electronic health monitoring systems. These may take the form of telehealth programs, coaching apps and mobile monitoring systems.

In eHealth solutions, such as telehealth programs and coaching apps, the patient (i.e., user) is often required to interact with the system to receive important learning content. For example, patients may receive learning content such as instructional videos on how to use therapy devices, health brochures and therapeutic device manuals, as well as disease and medication-related information.

If patients do not receive this learning content, they will fail to acquire the right knowledge and skills to meet learning goals defined by the service, which in turn may increase their likelihood of being non-compliant with their therapy regime. For example, they may not have an appropriate understanding and acceptance of their therapy and medication, or they may not know how to correctly perform the therapy or intervention needed to manage their disease.

Acquiring the right knowledge and skills are therefore an important part of the effective support for patient self-management/disease management. One critical factor affecting patients in meeting learning goals is that they are sometimes not receptive to learning interventions because they are not in a suitable affective and/or mental state geared for learning, i.e., they are not in a learning mode or have cognitive processing availability.

For example, they may be performing a cognitively light mental task such as browsing social media when they are prompted by a learning intervention to perform a task demanding heavy cognitive processing such as reading an informational brochure on the mechanism of action of bronchodilator medication. This mismatch between the learning mode of the patient and cognitive demands of the content in the learning intervention may increase the likelihood that the intervention fails to engage the user and support them in consuming or processing their learning material.

In order to make patients more receptive to learning interventions, the learning content should thus fit their mental state. It is well known that people's mental state is important for learning, for example this realization is used in the elaboration likelihood model in cognitive psychology. In this model, a user's mental state (positive or negative) influences their attention, which impacts the user's ability to process certain information. For instance, when a user is in a negative state they may process certain information with low attention, which in the elaboration likelihood model is labelled as a "low motivation and ability to process information". This leads to superficial processing of the information, resulting in only a temporary change that is susceptible to fading, which in a learning context would lead to lack of or incomplete retention of the knowledge or skill.

There are three basic ways to match an individual's receptiveness to learning content. These are to adapt the depth of content, the communication agent and the nature of the content delivery (i.e., language complexity and tone). If a learning intervention matches a user's mental state, this will increase the likelihood of successful and efficient transfer of the necessary learning information.

The patient's affective or emotional state is a highly relevant factor when considering their mental state in the context of their receptiveness to learning content. It has been reported in the literature that affective states influence patient engagement and compliance, i.e., in a negative state, people are less committed to continuing with and completing an intervention. It has indeed been reported that positive psychological states are linked to superior cardiac outcomes, possibly mediated through increased participation in health behaviors.

One approach could be to ask a user/patient what type of learning content they would prefer at a given moment. However, this is time consuming, burdensome to the user, and is not guaranteed to lead to the successful acceptance and consumption of learning material.

There is therefore a need for a system which can deliver the most appropriate learning content fitting the patients mental state, which leads to the most efficient content transfer.

### SUMMARY OF THE INVENTION

The invention is defined by the claims.

According to examples in accordance with an aspect of the invention, there is provided a user interface system, comprising:
an input for receiving activity information relating at least to electronic device usage by the user;
an output for providing learning content to a user, wherein the learning content is associated with a set of learning goals each having an associated difficulty level;
a processor for deriving a mental state of the user based on the activity information received during a preceding finite time period;
a database structure storing:
   the learning content;
   first information about the learning goals of the learning content and their associated difficulty levels, and whether or not those goals have been met; and
   second information about the mental state of the user; and
a content selection unit, which is adapted to select suitable learning content for output to the user based on the first information and the second information, such that suitable learning content is selected based on the mental state of the user before the commencement of delivery of the learning content.

This system selects learning content to be presented to a user based on a derived preceding mental state of the user and the learning goals which are being followed. The mental state is derived from the activity information before the learning content delivery starts. Thus, rather than using the delivery of the learning content itself to ascertain a mental state of the user, the mental state is obtained from other activity information in advance of the start of content delivery. This activity information thus relates to the user behavior immediately preceding the planned delivery of leaning content.

This preceding time period may typically be 30, 45 or 60 minutes or may be longer or shorter depending on time of day, duration of device usage and user location.

The learning goals are for example set by a healthcare provider or the patient or the self-management support system, grouped according to whether they are completed or incomplete at a given moment in time. These learning goals may or may not be explicit to the user.

The content selection unit processes the learning goals and the user's mental state (the second information) which is based on the activity information. Based on the processing of these inputs, a most suitable element of learning content, or a list of suitable learning content elements, may be determined for the next planned learning intervention, which best fits the user's mental state and the targeted learning goal(s) of the user. The learning content is selected also based on the progress of the user towards achieving the target learning goals, hence accounting for the remaining incomplete learning goal(s). The content selection unit does not simply select the learning content that best matches the user's mental state as this may include content related to learning goals that the user has previously completed. For example, if the user is only lacking more complicated knowledge, they will not receive completed easier learning content.

The activity information may be an aggregation of information, missing out periods of inactivity of the user. Thus, the preceding time period may be a combined time period during which activity information is collected rather than a continuous time period.

One or more learning goals of the learning content may comprise a set of sub-goals, wherein each sub-goal has a different learning difficulty. Thus, within a given learning goal, there may be different training elements requiring different levels of concentration.

The content selection unit may be part of the processor or it may be a separate processor.

The activity information for example relates to one or more of:
app usage by the user;
types of electronic device which have been used by the user;
online interaction by the user.

This activity information may be obtained from sensors in a user's device such as the touchpad, camera, accelerometer, gyroscope, etc. This may include information such as scroll-speed, eye movement, device orientation, contact/interaction time with device, etc.

App and online behavior data may be obtained from user commands (e.g., the content of searches, contents of chats, hashtags, emoticons, etc.) and from website and app metadata (e.g., app or website name, page/e-book title, meta descriptions, etc.) and search engine optimization metrics (Alexa Ranking, PageRank, TrustRank etc.).

The learning content may comprise a set of elements, each categorized with a respective output modality. These output modalities for example comprise video, audio, image, text, etc. Different modalities may be suitable for different mental states.

The elements of the learning content are for example each categorized with different characteristics relating to one or more of: the depth of content; the communication agent; the tone; and the language complexity.

In this way, different elements of the learning content may be selected which best match the learning goals still to be met and the current mental state.

Content depth characteristics for example include page count, word count, word count per page, graphics count, graphics count per page, time duration/length, level of detail, graphics to text ratio, file size, etc.

Communication agents include avatars, emoticons, professional agents such as doctors, non-serious agents such as clowns, or peer agents such as a fellow patient.

Content language complexity and tone for example relate to pitch, cadence and tone (of audible & written/visual content). This includes categorizations such as scientific, authoritative, directive, technical, collaborative, social, supportive, competitive, factual, action-oriented, etc.

The database structure may further comprise historical information relating to historical activity information and compliance data of the user, thereby providing information about the effectiveness of previous selected content.

The system can then adapt and learn based on the success or otherwise of delivering particular types of content when the user is determined to have a particular mental state. This compliance may be determined automatically for many task, because the user uses the user interface system to perform the task, such as playing or reading content.

The processor may be adapted to:
determine a dominant mental state based on a ratio ρ= τₛₑᵣᵢₒᵤₛ / τₙₒₙ₋ₛₑᵣᵢₒᵤₛ between the duration of device interaction and app/online behavior that is more serious to that which is less serious; and
use the ratio to determine if the user is in a serious mental state or a non-serious mental state.

This enables a simple binary measure to be provided of the user's mental state, in terms of their ability to process complicated subject matter.

The processor may be further adapted to determine a user attention level from the activity information. The user attention may for example be assessed based on the front camera images of the device on which the user consumes the learning content. The less the user looks at the screen, the lower the attention is rated. The content selection unit may then select content that assists in increasing motivation and the ability to process information.

Steps may be taken to increase the user attention, for example by sending messages or images that are likely to grab the attention of the user.

The processor may be further adapted to determine a user mental fatigue from the activity information. For example, if during a prolonged period of time, the user has spent most time engaged in learning mode with a limited number of activities, the system may conclude that the user is hyper focused and thereby could easily become fatigued with additional complex information.

The database may further comprise information relating to the user's personal activity agenda. This provides another way to know the likely activity type of the user, in addition to sensing in real time. Thus, it can be used to predict when a sudden change is activity level or type is expected.

The system may comprise an input for receiving location information from a portable device of the user. This can also be used to determine what types of content are suitable for delivery to the user. It can also be used in conjunction with the personal agenda (mentioned above) to verify that the personal agenda is being followed.

The invention also provides a user interface method, comprising:
receiving activity information relating at least to electronic device usage by the user;
deriving a mental state of the user based on the activity information received during a preceding finite time period;
selecting suitable learning content for output to the user, wherein the learning content is associated with a set of learning goals each having an associated difficulty level, wherein the selecting is based on:
   first information about the learning goals of the learning content and their associated difficulty levels, and whether or not those goals have been met; and
   second information about the mental state of the user; and
providing the selected learning content to the user, such that suitable learning content is selected based on the mental state of the user before the commencement of delivery of the learning content.

The method may comprise collecting activity information which relates to one or more of:
app usage by the user;
the type of electronic device used by the user;
online interaction by the user.

Selecting the most suitable learning content may further take account of historical information relating to historical activity information and compliance data of the user. The method is in particular adapted to provide health learning content to a user.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a user interface system; and
Figure 2 shows a user interface method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a user interface system which receives activity information relating at least to electronic device usage by a user. A mental state of the user is derived from this the activity information, received during a preceding finite time period. Learning content is provided to a user based on learning goals and their associated difficulty levels, based on whether or not those goals have been met and also based on the mental state of the user. In this way, suitable learning content is selected based on the mental state of the user before the commencement of delivery of the learning content.

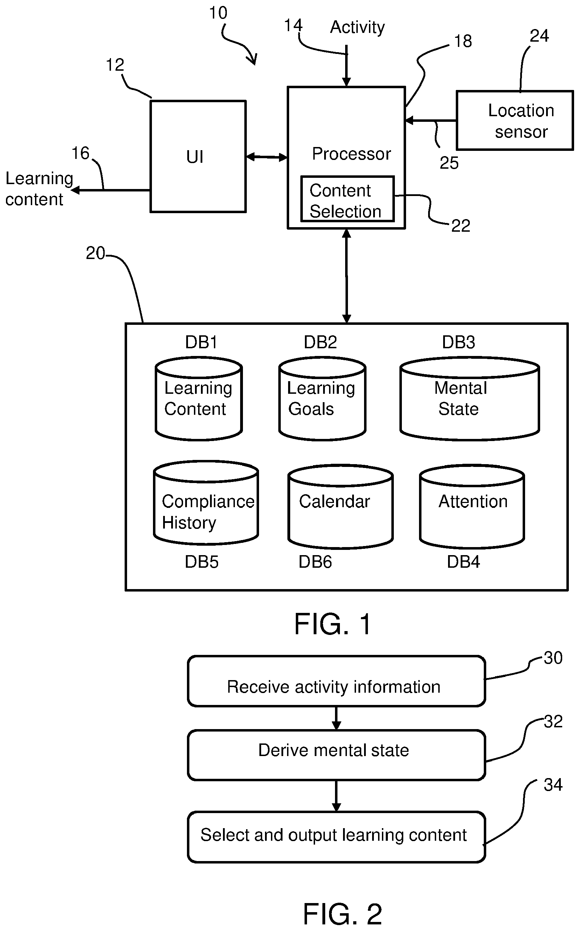

Figure 1 shows a user interface system 10, comprising an input 14 for receiving activity information relating at least to electronic device usage by the user.

The system has a user interface (input/output) device 12 for example a mobile phone on which an app is loaded. The user interface device 12 generates an output 16 to the user, which is learning content for the user. It may be provided as a displayed image or video, a text message, an audio output or indeed any suitable way of delivering content to the user.

The activity information is provided to a processor 18 of the system. The activity information may be derived from user activities (e.g. web browsing) using the user interface device 12 or it may be derived from sensors forming part of the user interface device 12 (accelerometers, cameras, microphones etc.) or it may be derived from other sensors not forming part of the user interface device. Thus, the activity information may be an external input to the system, but it may instead or additionally be information derived by the user interface device 12.

The activity information relates to any activities of the user which can be tracked electronically. For example, the information relates to apps that are being used by the user on the device which also implements the user interface system. The device is for example a smart phone or tablet of the user. The activity information may also relate to the types of electronic device which have been or are being used by the user (such as mobile phones, tablets etc.), as well as the online interaction by the user.

This activity information may be obtained from sensors in a user's device such as the touchpad, camera, accelerometer etc. This may include information such as scroll-speed, eye movement, device orientation, contact/interaction time with device, etc.

App and online behavior data may be obtained from user commands (e.g., the content of searches, contents of chats, hashtags, emoticons, etc.) and from website and app metadata (e.g., app or website name, page/e-book title, meta descriptions, etc.) and search engine optimization metrics (Alexa Ranking, PageRank, TrustRank etc.).

The activity information is processed by the processor 18, in order to derive a mental state of the user, in particular based on the activity information received during a preceding finite time period. This time period may be 30, 45 or 60 minutes or may be longer or shorter depending on time of day, duration of device usage and location. The activity information may be an aggregation of information, missing out periods of inactivity of the user. Thus, the preceding time period may be a time period which combines non-continuous time segments during which activity information is collected rather than a continuous time period. For example, user device interaction is typically intermittent, i.e., in blocks of focused device interaction interrupted by other non-device related daily activities (e.g., bio-breaks, food preparation, food consumption, etc.). The content selection unit can thus aggregate the device interaction blocks in the preceding time period to derive the user mental state.

The output 16 is used to provide selected learning content to the user. The learning content is associated with a set of learning goals each having an associated difficulty level.

In order to select the most appropriate learning content, the processor 18 has a content selection unit 22. It uses information stored in a database structure 20. The database structure is shown in Figure 1 as storing the learning content as content DB1. However, the learning content may be stored locally, or it may be a resource which is accessed remotely. Thus, the database structure 20 may be distributed over different locations, with some content local to the device of the user and some remote, and accessed over the Internet.

The database structure also includes first information DB2 about the learning goals associated with different elements of learning content and their associated difficulty levels, and it also records whether or not those goals have been met by the particular user.

The database structure also includes second information DB3 which stores the mental state of the user, as derived by the processor 18.

The learning content is selected based on the first information and the second information, such that suitable learning content is selected based on the mental state of the user before the commencement of delivery of the learning content.

In particular, the content selection unit 22 selects learning content matching both the user's mental state and their progress towards achieving the target learning goals of the learning content. There may be overall learning goals and sub-goals, and different sub-goals may have different difficulty levels. Thus, a learning goal may be broken up into smaller sub-goals of different levels of learning difficulty for the user and these sub-goals can be achieved over a finite time period, such as days, weeks or months, etc.

The learning content may be stored in the database structure with multiple modalities (e.g., video, audio, image, text, etc.) and also with different characteristics for example related to: i) depth of content, ii) communication agent(s) and iii) tone and language complexity.

Content depth characteristics include page count, word count, word count per page, graphics count, graphics count per page, time duration/length, level of detail, graphics to text ratio, file size, reading level, etc.

Communication agents may include avatars, emoticons, professional agents such as doctors, playful agents such as clowns, or peer agents such as a fellow patient.

Content language complexity and tone pertains to the pitch, cadence and tone of audible, written, and visual content. For example, tone includes qualitative aspects of the learning content which relate to whether the content is scientific, authoritative, directive, technical, collaborative, social, etc.

Depending on the user's progress in meeting their targeted learning goal(s) or sub-goal(s), all or only a subset of the learning content may be relevant for the user in the upcoming learning intervention. The content selection unit selects the most suitable learning content matching the user's mental state, e.g., learning mode, non-serious mode, etc., while accounting for the remaining incomplete learning goal(s) or sub-goal(s). In short, the content selection unit selects the learning content which matches the user's mental state while also allowing the user to make progress towards achievement of their learning goal.

The system thus selects learning content to be presented to a user based on a derived preceding mental state of the user and the learning goals which are being followed. The mental state is derived from the activity information before the learning content delivery starts. Thus, rather than using the delivery of the learning content itself to ascertain a mental state of the user, the mental state is obtained from other activity information in advance of the start of content delivery. This activity information thus relates to the user behavior immediately preceding the planned delivery of leaning content.

The user device interaction and app and online behavior can be a mixture of both more serious and less serious (i.e. playful) activity during consecutive and/or overlapping periods of time. For this reason, the processor may determine a dominant mental state based on a ratio p= τₛₑᵣᵢₒᵤₛ / τₙₒₙ₋ₛₑᵣᵢₒᵤₛ between the duration of device interaction and app/online behavior that is more serious to that which is less serious.

The ratio is then used to determine if the user is in a serious mental state or a non-serious mental state. This enables a simple binary measure to be provided of the user's mental state, in terms of their ability to process complicated subject matter.

If the ratio is more than 0.5 then the user will be determined to be in a more serious mental state while if it is less than 0.5 the user will be deemed to be in a more non-serious state. In the unlikely event that r equals 0.5, the time duration may be increased or decreased until the user's binary mental state can be clearly distinguished.

To illustrate the operation of the system, a first exemplary situation may be considered in which a COPD patient has target learning goals of: i) understanding and accepting their non-invasive (NIV) therapy and bronchodilator medication; and ii) knowing how to correctly perform the NIV therapy.

They have previously read a basic infographic summarizing key information on bronchodilators and watched a video clip of a doctor in a white coat showing the correct use of an inhaler to deliver bronchodilation medication.

To meet their learning goals for the week they are required to learn about: i) the mechanism of action of their bronchodilator medication; ii) the correct wearing of the ventilation mask and operation of their NIV therapy device; and iii) NIV therapy and its importance in COPD disease management.

At a given moment in time on a particular day when the patient is at home, after they have been using their IPAD for the previous hour to shop online for shoes, check Facebook and video call with a friend, their next learning intervention is planned.

The content selection unit will analyze their previous app and online behavior to determine that their mental state is less serious and more non-serious, i.e. playful, based on their device interaction and online/app behavior in the previous hour. It will then select from the remaining incomplete learning sub-goals and the learning content database, learning content which matches their non-serious mental state.

The content selection unit will for example produce as output a recommendation of the top three learning content offerings that will likely achieve the most efficient content transfer to the patient at that moment in time based on their target learning goals. In this situation, the content selection unit will recommend that the patient:
i) watch a video from a peer patient or informal avatar showing how to wear the ventilation mask and use the NIV therapy device; or
ii) receive an easily digestible infographic summarizing the correct steps to follow when wearing a ventilation mask and performing NIV therapy; or
iii) listen to a short podcast about NIV therapy for COPD disease management delivered in a down-to-earth and perhaps humorous manner by a fellow patient.

In this way, the patient will make further and efficient progress towards their target learning goals for the week, and will be more likely to follow through with the learning intervention since the content selection unit will select learning content that matches their mental state.

A second exemplary situation may be considered in which another patient with the same learning goals has spent two hours in the afternoon, intermittently using their tablet to read an e-book novel and watch the news, and their smartphone to communicate with family on WhatsApp and manage their bank account. They have also previously watched a video clip showing a fellow patient demonstrating the correct wearing and use of a NIV mask, and read a scientific article on the mechanism of action of bronchodilators.

To meet their learning goals for the week they need to: learn about: i) the correct use of an inhaler to deliver bronchodilation medication; ii) NIV therapy and its importance in COPD disease management; and iii) when and how often to use their bronchodilator medication. At a given moment in time later in the afternoon their next learning intervention is planned.

The content selection unit will analyze their previous app and online behavior to determine that their mental state is generally more serious (i.e., ρ>> 0.5) based on aggregating their intermittent device interaction and online/app behavior in the previous two hours. It will then select from the remaining incomplete learning sub-goals and the learning content database, learning content which matches their more serious, learning-oriented mental state.

The content selection unit will produce as output a recommendation of the top three learning content offerings that will likely achieve the most efficient content transfer to the patient at that moment in time based on their target learning goals. In this situation, the content selection unit may for example recommend that the patient:
i) read an article on bronchodilator medication use by COPD patients written in a scientific magazine-like tone, clearly explaining how it works;
ii) watch a video presented by a doctor wearing a white coat in an explaining NIV therapy and its mechanisms in COPD disease management; or
iii) read a technical inhaler manual explaining how to correctly use an inhaler to deliver bronchodilator medication, and commonly made errors.

In this way the patient will make further and efficient progress towards their target learning goals for the week, and will be more likely to follow through with the learning intervention since the content selection unit will select learning content that matches their mental state.

A user's mental state (positive or negative) will influence their attention - i.e. when in a negative state the user may process certain information with low attention. In the elaboration likelihood model this is labelled as a "low motivation and ability to process information". This leads to superficial processing of the information, resulting in only a temporary change that is susceptible to fading, which is undesired in the current context.

There are several strategies that can be applied to increase a user's motivation and ability to process information. This would lead to deeper processing and an increased focus of the user on the quality of the message arguments, resulting in lasting change (i.e., learning) that resists fading.

Options to increase user motivation to process the information would be to utilize learning content that:
appeals to intrinsic needs of the user, i.e. related to personals goals (e.g. being able to walk short distances, play with grand kids, be out of breath less often);
uses new types of stimuli (e.g. different font, size);
uses prominent stimuli (e.g. large pictures, bright colors);
uses a degree of complexity to invoke curiosity (e.g. by using interactive images to uncover complex structures, such as the lungs).

There are various ways to increase the ability of the user to process the learning content that is provided. Related knowledge structures of the user can be triggered (by relating the new content to something the user already knows or recognizes) by using verbal labels to refer to a different context/framework.

New knowledge can be created by giving concrete examples on how to use this information to reduce COPD related symptoms. Demonstrations may be used that encapsulate the learning content (e.g. by using animations or videos that vividly describe the learnings, such as how an exacerbation works). Analogies may also be used.

The user attention level may also be assessed by processing some of the activity information to generate attention level information DB4. For example, images may be acquired by the front camera of a mobile phone device on which the user consumes the learning content. The less the user looks at the screen, the lower the attention is rated. The content selection unit may for example select information types that aim to increase motivation and hence the ability to process information.

To illustrate this concept, a situation may be considered in which a COPD patient is determined to have a low attention level and they are about to receive content related to the correct use of bronchodilator medication. To increase the attention level of the user, the content selection unit may in this case decide to:
i) change the font type, size and color of the "correct use of bronchodilator medication" content; and/or
ii) show a splash screen before showing the actual content, depicting the intrinsic needs of a user (e.g. "Remember that you stated you want to be able to play with your grandchildren again? The following content will help you on your journey towards doing that"); and/or
iii) invoke curiosity by using complex images or adding an inviting title ("Using bronchodilator medication is easy? I think not!").

After execution of one or more of these adaptations by the content selection unit, the processor can assess whether the user indeed has increased attention as a consequence of these adaptations. Thus, there is a feedback loop in place. Ideally, the content selection unit will start with single adaptations to identify which is most effective. If none of the adaptations are effective on their own, the content selection unit can try to combine adaptations to identify what is most effective for a user.

In some situations, such as when a user has spent significant time within a serious mental state or in learning mode (e.g., having had a more than average strenuous work day), the preceding activity information may reliably reflect the user's current mental state, it may be beneficial to take account of possible mental fatigue. Thus, a mental fatigue threshold may be introduced. When this mental fatigue threshold is exceeded, no serious, technical, long or complex learning content should be deployed to the user.

A mental fatigue threshold can be derived from the activity information. If during a prolonged period of time, such as two hours, the user has spent most time engaged in learning mode with a limited number or activities (for example at most three), the system may conclude that the user is focused on one particular task and is hyper focused. Straining the user further with complex learning material may cause loss of focus and add to the user's cognitive load, leading the user to deplete their cognitive resources and reach mental fatigue earlier in the day than without the learning intervention.

To illustrate this concept, a user may be looking up literature on a certain topic for an hour using two or three scientific online databases such as PubMed, IEEE and Google Scholar. She then switches to the task of processing found material (highlighting pieces of text, copy-pasting, typing, deep linking) for another hour. We may conclude that the user is in a learning mode, but also in a hyper focused mode. The system should not disrupt the focus and add to the cognitive load, as the goals of acceptable and efficient data transfer may not be met if the system side tracks the user.

In some situations, such as when a user just wakes up, their mental state may be hard to assess due to limited availability of preceding device interaction data. In such a situation, the preceding time period used to assess the mental state may be automatically and dynamically adapted. For example, the time period may be reduced from 30, 45 or 60 minutes to 5, 10 or 15 minutes or an arbitrary time period to allow sufficient user interaction data to be collected.

Historical user intervention compliance and device interaction data may also be stored in the database structure, shown as information DB5 in Figure 1.

For some types of learning goal, it is possible to determine automatically and directly whether or not the user finished the task at hand. For example, it can be determined if the user read electronically delivered content by assessing if the task prompt was opened, if the user scrolled through to the end, or if the user pressed a "next" command the correct number of times. The user may also provide an input to the user interface system to confirm completion of a task.

Indirect methods are also possible, for example based on detecting changes in the user's behavior after delivery of an instruction message, for example looking up more information (e.g. healthy recipes), becoming more active, changing a bed time etc. Other sources may also detect changes which result from adherence to a task (e.g., increased pick up rate for medication, reduced hospitalization, etc.).

Some examples of possible tasks and ways to determine compliance are:
i) A user receives educational audio, video or a podcast. The compliance can be determined based on the content being played until the end, based on automatic monitoring by the user interface system;
ii) A user receives an educational text message. The compliance can be determined based on whether the user opened the message and scrolled to the bottom of message, and may again be based on automatic monitoring by the user interface system;
iii) A user receives a reminder to collect medication. The compliance is based on determining that the user has collected the medication on time, based on automatic input from the patient medical record;
iv) A user is required to take a dose of medicine. The compliance can be determined based on whether the medication has been taken, e.g. by using a smart nebulizer which confirms that the device has been used correctly (e.g., duration of inhalation) or by using a smart pill box which monitors when the patient takes medication;
v) A user is required to call or chat with a GP/coach using a care platform on a tablet. Compliance can be based on data logged automatically by care platform.

The compliance data allows the system generally to learn about a particular user's response patterns to particular tasks when they have a particular mental state. The compliance data can also be used to provide an additional input to the content selection unit to augment a limited amount of preceding device interaction data.

To illustrate this concept, a COPD patient may be considered, who wakes up in the morning and interacts with their smartphone to check the weather, the local news and their work email over a fifteen-minute period. In this case, the content selection unit will recognize that there is limited preceding device interaction data due to the COPD patient being previously asleep, and will automatically reduce the preceding time period to five minutes to allow sufficient device interaction data to be acquired to determine the patient's mental state.

If after five minutes, insufficient data is collected for the content selection unit to determine the patient's mental state, the time period may be extended to 10 or 15 minutes. In the event that the patient's device interaction is very limited in the wake up period, data on user intervention compliance and effectiveness during this time period using different types of learning content may be utilized to augment the device interaction data. For example, if in the past the patient successfully met a learning goal/sub-goal in the morning by watching a video delivered by a clinician with detailed scientific information explaining mechanism of action of bronchodilators, then the content selection unit may select more detailed, serious and technical content and communication agents instead of light, playful and non-serious content.

In some situations, such as when a user shifts from one setting to another (e.g., from work to a home or vice versa, or from home to the car), the preceding device interaction data may not reliably reflect the user's current mental state. Location data 25 from a location sensor may then be used (such as GPS signal, Wi-Fi, etc.) and/or information DB6 from a user's calendar may be used to identify moments at which a user's mental state may be transitioning sharply.

In these situations, the preceding device interaction user data may be ignored by the content selection unit or assigned a lower weighting when selecting the most appropriate content to meet an education goal or sub-goal. As explained above, historical user intervention compliance and device interaction may be used to replace the unreliable preceding device interaction data.

To illustrate this concept, a situation may be considered in which a COPD patient comes home by car from work in the evening. In the preceding two hours, the patient's device interaction may include the surfing the web on their laptop for work related technical information, listening to a podcast on personal development in their car ride home, and messaging their spouse via WhatsApp before reaching home. In this case, the content selection unit will ignore or decrease the weight of the prior device interaction data and place more emphasis on the patient's device interaction after their arrival home when they may be more relaxed.

In this way, an inappropriate learning content selection may be avoided which might result in the patient receiving more serious, technical, and detailed content based on the preceding time period in which device interaction data pertaining to their job may not reflect their current mental state.

Figure 2 shows a user interface method, comprising:
in step 30, receiving activity information relating at least to electronic device usage by the user;
in step 32, deriving a mental state of the user based on the activity information received during a preceding finite time period; and
in step 34, selecting and outputting suitable learning content for output to the user, wherein the learning content is associated with a set of learning goals each having an associated difficulty level. The selecting is based on the learning goals of the learning content and their associated difficulty levels, and whether or not those goals have been met, and the mental state of the user.

As discussed above, the system makes use of a processor to perform the data processing. The skilled person would be readily capable of developing a processor for carrying out any herein described method. Each step of a flow chart may represent a different action performed by a processor, and may be performed by a respective module of the processor.

The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A user interface system, comprising:
an input (14) for receiving activity information relating at least to electronic device usage by the user;
an output (16) for providing learning content to a user, wherein the learning content is associated with a set of learning goals each having an associated difficulty level;
a processor (18) for deriving a mental state of the user based on the activity information received during a preceding finite time period;
a database structure (20) storing:
the learning content (DB1);
first information (DB2) about the learning goals of the learning content and their associated difficulty levels, and whether or not those goals have been met; and
second information (DB3) about the mental state of the user; and
a content selection unit (22), which is adapted to select suitable learning content for output to the user based on the first information and the second information, such that suitable learning content is selected based on the mental state of the user before the commencement of delivery of the learning content.

2. The system of claim 1, wherein the activity information relates to one or more of:
app usage by the user;
types of electronic device which have been used by the user;
online interaction by the user.

3. The system of claim 1 or 2, wherein the learning content comprises a set of elements, each categorized with a respective output modality.

4. The system of claim 3, wherein the elements of the learning content are each categorized with different characteristics relating to one or more of: the depth of content; the communication agent; the tone; and the language complexity.

5. The system of any one of claims 1 to 4, wherein the database structure further comprises historical information (DB4) relating to historical activity information and compliance data of the user, thereby providing information about the effectiveness of previous selected content.

6. The system of any one of claims 1 to 5, wherein the processor is adapted to:
determine a dominant mental state based on a ratio ρ= τₛₑᵣᵢₒᵤₛ / τₙₒₙ₋ₛₑᵣᵢₒᵤₛ between the duration of device interaction and app/online behavior that is more serious to that which is less serious; and
use the ratio to determine if the user is in a serious mental state or a non-serious mental state.

7. The system of any one of claims 1 to 6, wherein the processor is further adapted to determine a user attention level from the activity information.

8. The system of any one of claims 1 to 7, wherein the processor is further adapted to determine a user mental fatigue from the activity information.

9. The system of any one of claims 1 to 8, wherein the database further comprises information (DB5) relating to the user's personal activity agenda.

10. The system of any one of claims 1 to 9, comprising an input (25) for receiving location information from a portable device (24) of the user.

11. A user interface method, comprising:
receiving activity information relating at least to electronic device usage by the user;
deriving a mental state of the user based on the activity information received during a preceding finite time period;
selecting suitable learning content for output to the user, wherein the learning content is associated with a set of learning goals each having an associated difficulty level, wherein the selecting is based on:
first information about the learning goals of the learning content and their associated difficulty levels, and whether or not those goals have been met; and
second information about the mental state of the user; and
providing the selected learning content to the user, such that suitable learning content is selected based on the mental state of the user before the commencement of delivery of the learning content.

12. The method of claim 11, comprising collecting activity information which relates to one or more of:
app usage by the user;
the type of electronic device used by the user;
online interaction by the user.

13. The method of any one of claims 11 to 12, comprising selecting the most suitable learning content further taking account of historical information relating to historical activity information and compliance data of the user

14. The method of any one of claims 11 to 13 for providing health learning content to a user.

15. A computer program comprising computer program code means which is adapted, when said program is run on a computer, to implement the method of any one of claims 11 to 14.
